# EUROPÄISCHE PATENTSCHRIFT

(11) **EP 1 521 761 B1**
(45) Veröffentlichungstag und Bekanntmachung des Hinweises auf die Patenterteilung: **11.01.2006**
(21) Anmeldenummer: 03757039.7
(22) Anmeldetag: 06.06.2003
(51) Int. Cl.: C07H 11/04, C07H 13/04, A61K 31/70

(54) **CDG-THERAPIE MIT MANNOSE**
TREATMENT OF CONGENITAL DISORDERS OF GLYCOSYLATION (CDG) USING MANNOSE
TRAITEMENT DE TROUBLES CONGENITAUX DE LA GLYCOSILATION AVEC DU MANNOSE

(30) Priorität: 07.06.2002 DE 10225628
(43) Veröffentlichungstag der Anmeldung: 13.04.2005
(73) Patentinhaber: Marquardt, Thorsten, 48149 Münster (DE); SHS Gesellschaft für Klinische Ernährung mbH, 74074 Heilbronn (DE)
(72) Erfinder: MARQUARDT, Thorsten, 48149 Münster (DE); THIEM, Joachim, 22391 Hamburg (DE); RUTSCHOW, Synke, 22087 Hamburg (DE)
(74) Vertreter: Köster, Hajo
(86) Internationale Anmeldenummer: PCT/EP2003/005986
(87) Internationale Veröffentlichungsnummer: WO 2003/104247

(56) Entgegenhaltungen:
- RUTSCHOW, SYNKE ET AL: "Membrane-Permeant derivatives of mannose-1-phosphate" BIOORGANIC & MEDICINAL CHEMISTRY (2002), 10(12), 4043-4049, XP002259386
- KNERR, LAURENT ET AL: "Efficient synthesis of hydrophilic phosphodiester derivatives of lipophilic alcohols via the glycosyl hydrogenphosphonate method" TETRAHEDRON LETTERS (1998), 39(3/4), 273-274, XP004100940
- GRÜNEWALD, S. ET AL.: "Congenital disorders of glycosylation: a review" PEDIAT.RES., Bd. 52, Nr. 5, 2002, Seiten 618-624, XP009019434
- WARREN, C. D. ET AL.: "Chemical synthesis of dolichyl- alpha-D-mannopyranosyl phosphate and citronellyl-alpha-D-mannopyranosyl phosphate" BIOCHEM., Bd. 12, Nr. 25, 1973, Seiten 5038-5044, XP002259388
- MAYATEPEK, E. ET AL.: "Mannose supplementation in carbohydrate-deficient glycoprotein syndrome type I and phosphomannomutase deficiency" EUR. J. PEDIAT., Bd. 157, 1998, Seiten 605-606, XP002259389 in der Anmeldung erwähnt

## Beschreibung

Die Erfindung betrifft Mannose 1-Phosphat Derivate, diese enthaltende pharmazeutische und diätetische Mittel sowie die Verwendung dieser Derivate zur Behandlung von angeborenen Glykosylierungsstörungen.

Bei den angeborenen Stoffwechselerkrankungen besteht ein extremes Missverhältnis zwischen der sehr großen Zahl diagnostizierbarer und der sehr kleinen Zahl therapierbarer Erkrankungen. Bei den wenigen behandelbaren Stoffwechselerkrankungen kommen vornehmlich diätetische Maßnahmen zum Tragen, um bei Abbaustörungen (z. B. von Aminosäuren) die Substratzufuhr zu reduzieren. Bei Biosynthesestörungen sind die therapeutischen Möglichkeiten noch begrenzter, so dass sich nur wenige Erkrankungen überhaupt therapieren lassen.

Angeborene Glykosylierungsstörungen (Congenital Disorders of Glycosylation (CDG)) sind vererbte Stoffwechselstörungen, die bereits in der Kindheit zu schwerwiegenden Symptomen führen, wozu eine ausgeprägte mentale und statomorische Retardierung, Krampfanfälle, Cardiomyopathie und schwere Gedeistörungen zählen.

Von den zehn bekannten Erkrankungen der angeborenen Glykosylierungsstörungen können bisher nur zwei effektiv therapiert werden (CDG-lb und CDG-Hc).

Das CDG-Ia stellt eine der häufigsten angeborenen Glykosylierungsstörungen und wurde phenotypisch 1980 zum ersten Mal beschrieben, man vergleiche *Jaeken, J.; Vanderschueren-Lodevireyckx, M.; Casaer, P.; Snoeck, L.; Corbeel, L.; Eggermont, E.; Edckels, R. Pediatr. Res. 1980, 14,* 179. Diese Erkrankung beruht auf einem Defekt der Phosphomannomutase 2, einem zytoplasmatischen Enzym, das die Umwandlung von Mannose 6-Phosphat zu Mannose 1-Phosphat katalysiert. Das Mannose 1-Phosphat stellt die Ausgangssubstanz für die Herstellung von GDP-Mannose dar. Dieses Zuckernukleotid wird benötigt, um Mannose in Dolichol-verknüpfte Oligosaccharidketten einzubauen, die dann für die N-Glykosylierung von Proteinen verwendet werden.

Ein Mangel an Mannose 1-Phosphat führt zu einem Mangel an Dolicholverknüpften Oligosacchariden. Dies führt wiederum zu einer Unterglykosylierung neu synthetisierter Glykoproteine. Da N-Glykane für die Funktion vieler Glykoproteine eine essentielle Bedeutung haben, führt die generalisierte Unterglykosylierung zu Funktionsstörungen in vielen Fällen und damit zu einer schweren Multisystemerkrankung mit den oben beschriebenen schwerwiegenden Symptomen. Ein alternativer Biosyntheseweg existiert nicht. Die Substanz wird mangels eines entsprechenden Transporters in der Zellmembran nicht vom Extrazellulärraum aufgenommen. Obwohl sich die Hypoglycosilierung von Glycoproteinen in Fibroblasten durch Zugabe von Mannose zum Kulturmedium verringern lässt, schlugen bisher alle Versuche fehl, CDG-la Kinder erfolgreich zu behandeln, E. Mayatepek et al. in Eur.J. Pediatr. 157: 605-606 und in 1. Acta Paediatr. 86: 1138-1140.

Aufgabe der vorliegenden Erfindung ist es, einen Weg zur Behandlung von angeborenen Glykosylierungsstörungen und insbesondere von CDG-Ia aufzuzeigen.

Gelöst wird diese Aufgabe durch die Lehre der Ansprüche.

Bei den erfindungsgemäßen bereitgestellten Mannose 1-PhosphatDerivaten handelt es sich um Verbindungen, die hydrophob maskiert sind. Es wird angenommen, ohne an diese Erklärung gebunden zu sein, dass hydrophob maskierte Mannose 1-Phosphat Derivate in der Lage sind, die hydrophobe Zellmembran zu überwinden. Im Zytoplasma angekommen, können die Verbindungen dann durch zytoplasmatisch vorkommende Enzyme gespalten werden, so dass Mannose 1-Phosphat freigesetzt wird. Damit wird der eingangs genannte intrazytoplasmatische Mangel an Mannose 1-Phosphat ausgeglichen.

Gegenstand der Erfindung sind somit Mannose 1-Phosphat Derivate der folgenden allgemeinen Formel I. 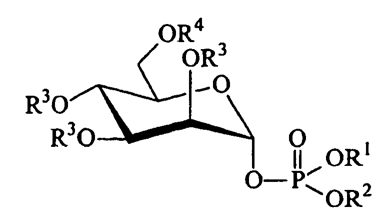 worin R¹ und R², die gleich oder verschieden sein können, für
H oder 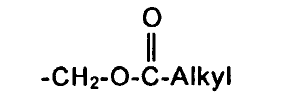 stehen,
oder R¹ und R² zusammen für: 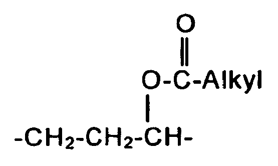 stehen,
mit der Maßgabe, dass nur einer der Reste R¹ und R² für H stehen kann, und
R³ und R⁴, die gleich oder verschieden sein können, für 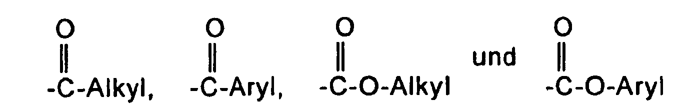 stehen
und der Rest R³ auch für H stehen kann, wobei
Aryl einen aromatischen Kohlenwasserstoffrest bedeutet, der durch einen Rest Alkyl substituiert sein kann und
Alkyl einen linearen oder verzweigten, gesättigten Kohlenwasserstoffrest mit 1 bis 20 Kohlenstoffatomen bedeutet.

Die Reste R1 und R2 können beispielsweise folgende Bedeutungsmöglichkeiten besitzen:
H, 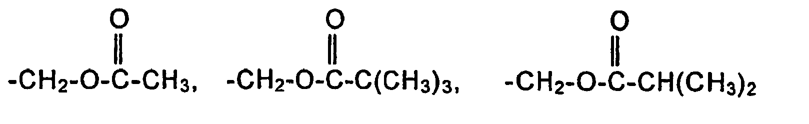
   und

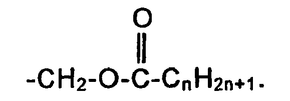

Ferner können R¹ und R² zusammen beispielsweise für folgende Reste stehen.

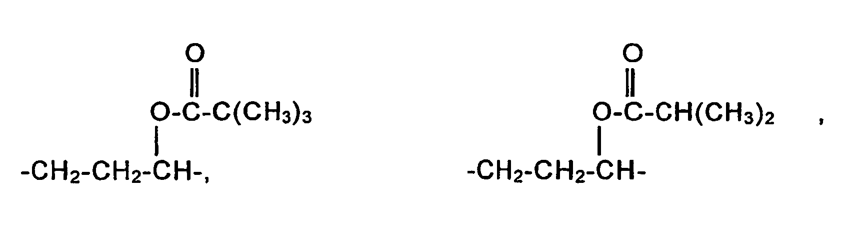

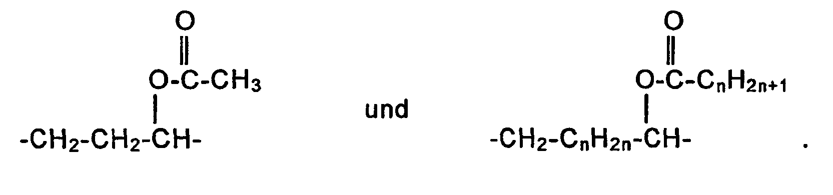

Der Index n steht dabei für 2, 3, 4, 5, 6, 7, 8, 9, 10, 11, 12, 13, 14, 15, 16, 17, 18 und 20.

Die Reste R³ und R⁴, die gleich oder verschieden sein können, stehen beispielsweise für
H, 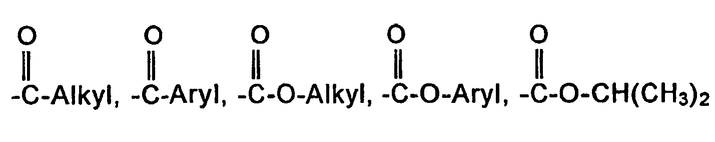 und

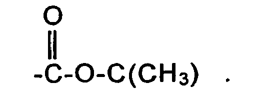

Vorzugsweise sind alle Reste R¹ und R² gleich. Weiterhin bevorzugt sind auch alle Reste R³ die gleichen. Weiterhin bevorzugt sind nicht nur alle Reste R³ als solche gleich, sondern auch der Rest R⁴ ist der gleiche wie die Reste R³.

Nach einer weiterhin bevorzugten Ausführungsform steht der Rest Alkyl bzw. die Alkylgruppe in einem oder mehreren der Reste R¹, R², R³ und R⁴ für CH₃-C(CH₃)₃, -CH(CH₃)₂ und -CH₂-CH₂-CH₃.

Bei dem Rest Aryl kann es sich beispielsweise um eine Phenyl- oder Napthylrest handeln, wobei dieser Rest durch einen, zwei, drei oder auch mehr Reste Alkyl gemäß der oben gegebenen Definition substituiert sein kann.

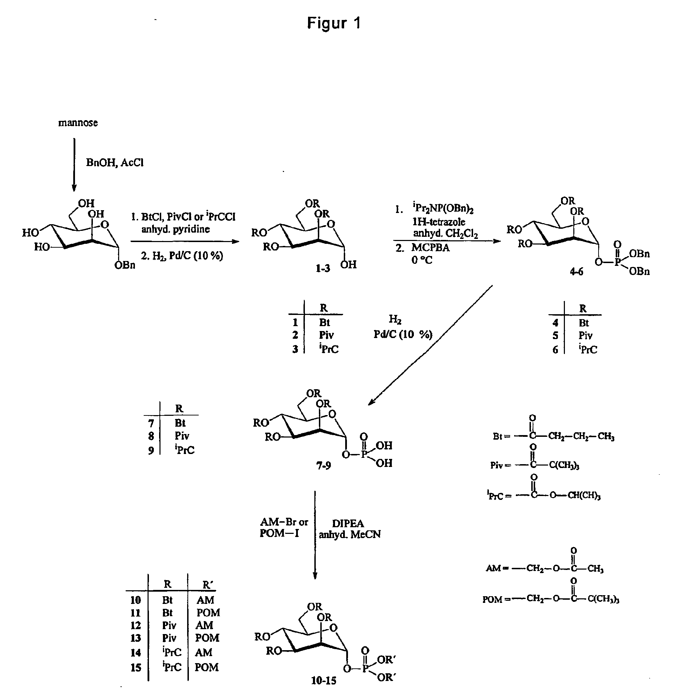

Die erfindungsgemäßen Mannose 1-Phosphat Derivate lassen sich nach dem in der Figur 1 dargestellten Syntheseweg herstellen. Ausgehend von Mannose wird Benzylmannopyranosid durch eine Fischer-Glykosylierung mit Benzylalkohol erhalten *(Dziewiszek, K.; Banaszek, A.; Zamojski, A. Tetrahedron Lett. 1987, 28, 1569*). Diese Verbindung wird in die in geeigneter Weise substituierten Mannopyranoside überführt, wobei Butyrylchlorid, Pivaloylchlorid oder Isopropylchlorformiat Anwendung finden *(Ogilvie, K. K.; Letsinger, R. L. J.Org. Chem. 1967, 32, 2365; Nicolaou, K. C.; Webber, S. E. Synthesis 1986, 453).* Durch anschließende Hydrogenolyse der Benzylgruppen auf Pd/C (10 %) werden die anomerisch ungeblockten Mannosederivate 1-3 erhalten.

Eine weitere Umsetzung mit Dibenzyl di-iso-Propylphosphoramidit unter Verwendung von 1H-Tetrazol ergibt die Phosphit-Triester, die in situ durch meta-Chlorperbenzoesäure (MCPCA) zu den entsprechenden Phosphatderivaten 4-6 oxydiert werden, *(Mills, S. J.; Potter, B. V. L. J. Chem. Soc., Perkin Trans. 1, 1997, 1279*). Anschließend werden die Benzylgruppen durch Hydrogenolyse auf Pd/C (10%) entfernt. Die erhaltenen Phosphate 7 - 9 werden in deren Acetoxymethyl- (AM) und Pivaloyloxymethyl-(POM)-ester 10 - 15 überführt, wobei Brommethylacetat oder lodmethylpivaloat in Anwesenheit von N-Ethyl-di-iso-propylamin (DIPEA) eingesetzt werden.

Obige Ausführungen und das in der Figur 1 gezeigte Syntheseschema erläutern die Herstellung der erfindungsgemäßen Verbindung anhand einiger bevorzugter Reste und der entsprechenden Reagenzien. Selbstverständlich können auch andere Reste und Reagenzien zur Anwendung gebracht werden, um die gewünschten Reste in das Mannose-Grundmolekül einzuführen.

Gegenstand der Erfindung sind auch pharmazeutische und diätetische Mittel, die als Wirksubstanz mindestens ein erfindungsgemäßes Mannose-1-Phosphat-Derivat enthalten. Es können somit 1, 2, 3, 4..... derartige Derivate vorhanden sein.

Das erfindungsgemäße Mittel kann ausschließlich aus einem erfindungsgemäßen Mannose-1-Phosphat-Derivat bestehen. In diesem Falle sind keine Hilfsstoffe, Träger, Adjuvantien etc. vorhanden. Letztere können jedoch ebenfalls in einem erfindungsgemäßen Mittel, sei es nun ein pharmazeutisches oder ein diätetisches Mittel vorhanden sein. Ferner können ein oder mehrere andere Wirkstoffe in das erfindungsgemäße Mittel inkorporiert werden.

Die erfindungsgemäßen Mittel lassen sich auf einfache Weise herstellen, beispielsweise durch Vermengen, Vermischen etc. und können in geeigneter Form verabreicht werden, beispielsweise als Pulver, als Tablette, als Kapsel und in jeder anderen geeigneten galenischen Form. Bei dem erfindungsgemäßen Mittel kann es sich auch um ein Diätetikum handeln. In diesem Falle werden die erfindungsgemäßen Mannose-I-Phosphat-Derivate beispielsweise einem Lebensmittel bzw. einem diätetischen Erzeugnis beigegeben. Dies kann beispielsweise auch im Rahmen einer Diät erfolgen.

Die erfindungsgemäßen Mittel dienen zur Behandlung von angeborenen Glykosylierungsstörungen und insbesondere zur Behandlung von CDG-Ia-Patienten. Die erfindungsgemäßen Mittel können auch dann Anwendung finden, wenn es erforderlich ist, hydrophob-maskierte Mannose-Derivate durch hydrophobe Zellmembranen zu "schleusen".

Gegenstand ist daher auch die Verwendung von Mannose-1-PhosphatDerivaten der allgemeinen Formel I, in der die Reste R¹, R², R³ und R⁴ physiologisch vorkommende Carbonsäuregruppen bilden, die über die OH-Gruppe des Mannose-Grundkörpers in Form von Estern daran gebunden sind, wobei R³ auch ein Wasserstoffatom bedeuten kann, zur Behandlung von angeborenen Glykosylierungsstörungen oder zur Herstellung von Mitteln zur Behandlung derartiger angeborener Glykosylierungsstörungen.

Bei diesen Carbonsäuregruppen handelt es sich vorzugsweise um kurze sowie physiologischerweise vorkommende Carbonsäuren.

Vorzugsweise werden Mannose-1-Phosphat-Derivate der allgemeinen Formel I verwendet, bei denen die Reste R¹, R², R³ und R⁴ die in den vorliegenden Unterlagen und in den Patentansprüchen konkret offenbarten Bedeutungsmöglichkeiten besitzen.

Die erfindungsgemäßen Mannose-1-Phosphat-Derivate dienen insbesondere zur Behandlung von CDG-Ia.

Zur weiteren Erläuterung bevorzugter erfindungsgemäßer Verbindungen wird auch auf die beiliegenden Figuren verwiesen. Dabei zeigen
- Figur 1: ein Syntheseschema in formelmäßiger Darstellung zur Herstellung der erfindungsgemäßen Mannose-1-Phosphat-Derivate, in dem diese Herstellung unter Bezug auf einige Vertreter der erfindungsgemäßen Mannose-1-Phosphat-Derivate beispielhaft dargestellt ist, und

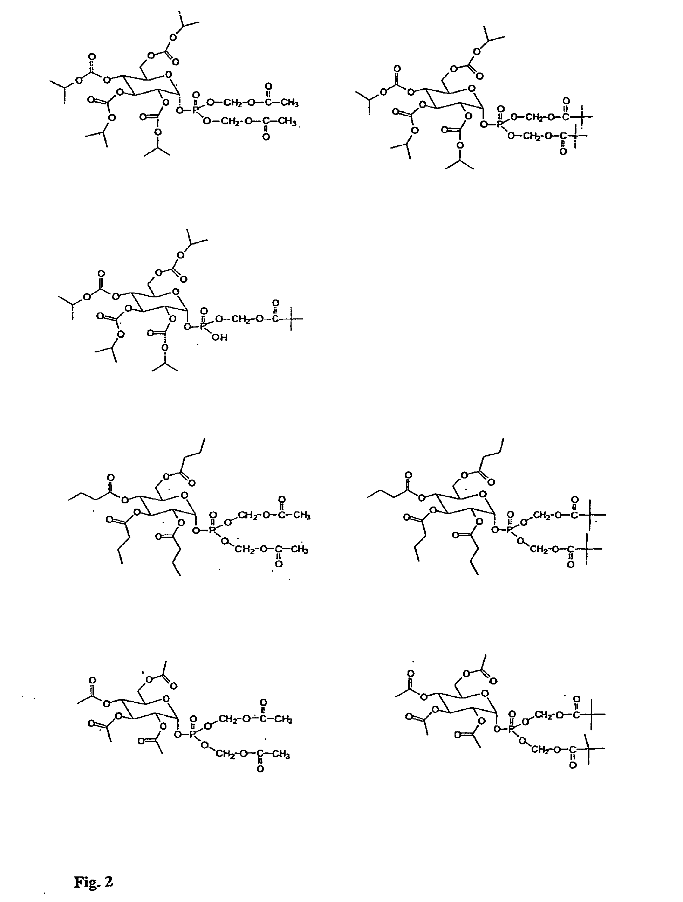

- Figur 2: Strukturformeln einzelner erfindungsgemäßer Mannose-1-Phosphat-Derivate; die konkrete Herstellung einige dieser Derivate ist in den nachfolgenden Beispielen näher beschrieben.

Nachstehend wird die Herstellung der erfindungsgemäßen Mannose-1-Phosphat-Derivate anhand bevorzugter Spezies erläutert.

Die NMR-Spektren wurden mit Bruker AC-250, AMX-400 und DRX-500 aufgenommen. Die chemischen Verschiebungen für ¹H NMR und ¹³C NMR sind bezüglich Tetramethylsilan angegeben. 85 %-ige Phosphorsäure wurde als externer Standard für ³¹P NMR eingesetzt. Die optische Drehung wurde mit einem Perkin-Elmer-Polarimeter 341 bestimmt. Die Schmelzpunkte wurde mit ST-Apotec gemessen und sind nicht korrigiert. MALDI-TOF Spektren wurden auf Bruker Biflex III und ESI Spektra auf aHP Series 1100 MSD aufgezeichnet. Für die Dünnschichtchromatographie (TLC) wurden vorbeschichtete Platten eingesetzt, Silicagel 60 GF₂₅₄ (Merck). Die Detektion erfolgte durch Beobachtung unter UV-Licht bei 254 nm und durch Besprühen mit 10 % - iger ethanolischer Schwefelsäure und anschließendem Erhitzen. Die Säulenchromatographie wurde mittels Flash-Technik unter Verwendung von Silicagel 60 (230 - 400 mesh, 0,040 - 0,063 mm, Merck) durchgeführt.

lodmethylpivaloat wurde auf bekannte Weise synthetisiert.

### Schutz von Benzylmannopyranosid und Hydrierung

Benzylmannopyranosid wurde in trockenem Pyridin (0,1 M Lösung) bei 0 ° C gelöst. Butyrylchlorid (3 eq/OH), Pivaloylchlorid (3 eq/OH) oder iso-Propylchlorformiat (1,5 eq/OH, 1 M Toluol) wurden hinzugetropft. Die Mischung wurde über Nacht bei Raumtemperatur gerührt.

### Aufarbeitung für Butyrylchlorid und Pivaloylchlorid:

Die Umsetzung wurde mit Methanol gequencht. Dann wurde die Lösung konzentriert und zusammen mit Toluol bei vermindertem Druck destilliert. Der Rückstand wurde in Dichlormethan gelöst, zweimal mit gesättigter Natriumhydrogencarbonatlösung und einmal mit Wasser gewaschen, dann über Magnesiumsulfat getrocknet, filtriert, bei vermindertem Druck eingeengt und wiederum mit Toluol co-destilliert. Der rohe Rückstand wurde säulenchromatographisch mit Petrolether/Ethylacetat (1:1) gereinigt.

### Aufarbeitung für iso-Propylchlorformiat:

Die Mischung wurde mit Chloroform verdünnt, zweimal mit 1 M Chlorwasserstoffsäure und einmal mit Wasser gewaschen, dann über Magnesiumsulfat getrocknet, filtriert, bei vermindertem Druck konzentriert und mit Toluol co-destilliert. Der rohe Rückstand wurde säulenchromatographisch mit Petrolether/Ethylacetat (1:1) gereinigt.

Anschließend wurde in trockenem Methanol (0,1 M Lösung) und Pd/C (10 %) (vorsichtig hinzugeben) hydriert. Die Mischung wurde bei Raumtemperatur bei normalen H₂-Druck gerührt. Nach Reaktionsende wurde die Lösung über Celit filtriert, bei vermindertem Druck konzentriert und säulenchromatographisch mit Petrolether/Ethylacetat (3:1) gereinigt, wobei die Verbindungen 1, 2 und 3 erhalten wurden.

### 2,3,4,6-Tetra-O-butyryl-α-D-mannopyranose (1).

Die Verbindung 1 wurde auf die oben beschriebene Weise hergestellt.

Ausbeute: 0.85 g (1.85 mmol, 52 % bezüglich Mannose, farbloser Sirup); R_{f} 0.49 in 1:1 Petrolether/Ethylacetat; ¹H NMR (400 MHz, CDCl₃) δ 5.44 (dd, 1H, H-3), 5.37 (dd~t, 1H, H-4), 5.31 (dd, 1H, H-2), 5.24 (s, 1H, H-1), 4.27-4.16 (m, 3H, H-5, H-6a, H-6b), 3.12 (bs, 1H, OH), 2.42-2.16 (m, 8H, 4x-CO-CH₂-), 1.77-1.52 (m, 8H, 4x-CH₂-CH₃), 1.03-0.88 (m, 12H, 4x-CH₃); J_{1,2} = 2.0, J_{2,3} = 3.1, J_{3,4} = 10.2, J_{4,5} = 9.7 Hz; ¹³C NMR (100.62 MHz, CDCl₃) δ 173.4, 172.7, 172.5, 172.3 (C=O), 92.4 (C-1), 69.7 (C-2), 68.8, 68.6 (C-3, C-5), 65.7 (C-4), 62.2 (C-6), 36.1, 36.0, 35.9 (-CO-CH₂-), 18.5, 18.3, 18.2 (-CH₂-CH₃), 13.7, 13.6 (-CH₃); C₂₂H₃₆O₁₀ (460.52).

### 2,3,4,6-Tetra-O-pivaloyl-α-D-mannopyranose (2).

Die Verbindung 2 wurde auf die oben beschriebene Weise hergestellt. Ausbeute: 1.38 g (2.63 mmol, 34 % bezüglich Mannose, weiße Kristalle); mp 175.8 °C; R_{f} 0.23 in 3:1 Petrolether/Ethylacetat; ¹H NMR (400 MHz, CDCl₃) δ 5.53 (dd~t, 1 H, H-4), 5.46 (dd, 1 H, H-3), 5.28 (dd, 1 H, H-2), 5.19 (bs, 1H, H-1), 4.29 (ddd, 1 H, H-5), 4.22-4.13 (m, 2H, H-6a, H-6b), 3.17 (d, 1H, OH), 1.27, 1.24, 1.16, 1.12 (4xs, 36H, 4x-C(CH₃)₃); J_{1,2} = 1.8, J_{2,3} = 3.1, J_{3,4} = 10.2, J_{4,5} = 10.2; ¹³C NMR (100.62 MHz, CDCl₃) δ 178.3, 177.3, 176.7, 172.0 (C=O), 92.5 (C-1), 69.9 (C-2), 69.1 (C-3), 68.9 (C-5), 65.2 (C-4), 61.9 (C-6), 38.9, 38.8 (Cq, -C(CH₃)₃), 27.2, 27.2, 27.1 (-C(CH₃)₃); C₂₆H₄₄O₁₀ (516.63).

### 2,3,4,6-Tetra-O-iso-propylcarbonat-α-D-mannopyranose (3).

Die Verbindung 3 wurde auf die oben beschriebene Weise hergestellt.

Ausbeute: 0.73 g (1.39 mmol, 73 % bezüglich Mannose, farbloser Sirup); R_{f} 0.34 in 3:1 Petrolether/Ethylacetat; ; ¹H NMR (400 MHz, CDCl₃) δ 5.34 (s, 1H, H-1), 5.26-5.22 (m, 2H, H-2, H-3), 5.09 (dd-t, 1H, H-4), 4.93-4.80 (m, 4H, 4x-CH(CH₃)₂), 4.32-4.28 (dd, 3H, H-5, H-6a, H-6b), 1.34-1.26 (m, 24H, 8x-CH(CH₃)₂); J_{4,5} = 9.7 Hz; ¹³C NMR (100.62 MHz, CDCl₃) δ 154.3, 153.9, 153.6, 153.4 (C=O), 92.1 (C-1), 73.0, 72.9, 72.7, 72.4 (-CH(CH₃)₂), 72.5, 72.1 (C-2, C-3), 70.0 (C-4), 68.5 (C-5), 66.1 (C-6), 21.7, 21.7, 21.6 (-CH(CH₃)₂; C₂₂H₃₆O₁₄ (524.52).

### Phosphorylierung

1H-Tetrazol (5 eq) wurden unter Argonatmosphäre in trockenem Dichlormethan (20 ml) suspendiert. Nach Zugabe von Dibenzyl-di-iso-propylphosphoramidit (2,5 eq) wurde die Mischung bei Raumtemperatur 15 min. gerührt, um die Tetrazolid-Zwischenverbindung herzustellen. Danach wurde eine Lösung der Mannosederivate 1, 2 oder 3 in 20 ml trockenem Dichlormethan hinzugegeben, und die Mischung wurde weitere 3 h bei Raumtemperatur gerührt, bevor auf 0 °C abgekühlt wurde. MCPCA (3 eq) wurden hinzugerührt. Dann wurde 1 h kontinuierlich gerührt. Die Lösungsmittel wurden bei vermindertem Druck entfernt. Die Reinigung erfolgte auf säulenchromatographisch mit Petrolether/Ethylacetat (3:1, 2:1), wobei die Verbindungen 4, 5 und 6 erhalten wurden.

### Dibenzyl-(2,3,4,6-tetra-O-butyryl-α-D-mannopyranosyl)-phosphat (4).

Die Verbindung 1 (1,80 g, 3,92 mmol) wurde auf die oben beschriebene Weise umgesetzt.

Ausbeute: 2,51 g (3,48 mmol, 89 %, Sirup); [α]_{D} +13,7 (c 0,4, CHCl₃); R_{f} 0,45 in 1:1 Petrolether/Ethylacetat; ¹H NMR (400 MHz, CDCl₃) δ 7.40-7.30 (m, 10H, Ph), 5.62 (dd, 1H, H-1), 5.36 (dd~t, 1H, H-4), 5.31 (dd, 1 H, H-3), 5.26 (dd~t, 1 H, H-2), 5.12-5.09 (m, 4H, 2x-CH₂-Ph), 4.14 (dd, 1 H, H-6a), 4.03 (ddd, 1 H, H-5), 3.95 (dd, 1 H, H-6b), 2.40-2.19 (m, 8H, 4x-CO-CH₂-), 1.75-1.53 (m, 8H, 4x-CH₂-CH₃), 1.01-0.88 (m, 12H, 4x-CH₃); J_{1,2} = 1.5, J_{2,3} = 3.1, J_{3,4} = 10.2, J_{4,5} = 9.7, J_{5,6a} = 4.1, J_{5,6b} = 2.0, J_{6,6} = 12.2, J_{H-1,P} = 6.1 Hz; ¹³C NMR (100.62 MHz, CDCl₃) δ 173.1, 172.3, 172.1, 172.0 (C=O), 130.2, 129.8 (Cq), 128.8-128.0 (C_{arom.}), 95.3 (d, C-1), 70.5 (C-5), 70.0 (d, -CH₂-Ph), 69.9 (d, -CH₂-Ph), 68.6 (d, C-2), 68.2 (C-3), 64.8 (C-4), 61.4 (C-6), 36.0, 35.9, 35.8 (-CO-CH₂-), 18.4, 18.3, 18.2, 18.1 (-CH₂₋CH₃), 13.7, 13.6 (-CH₃); ²J_{C-1,P} = 4.8, 2x ²J_{CH2,P} = 6.1, ³J_{C-2,P} = 10.9 Hz; ³¹P NMR (101.26 MHz, CDCl₃) δ -1.97; Anal. ber. für C₃₆H₄₉O₁₃P (720.76): C 59.99, H 6.85; gefunden: C 60.01, H 6.74.

### Dibenzyl-(2,3,4,6-tetra-O-pivaloyl-α-D-mannopyranosyl)-phosphat (5).

Die Verbindung 2 (1.38 g, 2.63 mmol) wurde auf die oben beschriebene Weise umgesetzt.

Ausbeute: 1.43 g (1.84 mmol, 70 %, Sirup); [α]_{D} +22.1 (c 0.7, CHCl₃); R_{f} 0.52 in 1:1 Petrolether/Ethylacetat; ¹H NMR (400 MHz, CDCl₃) δ 7.38-7.33 (m, 10H, Ph), 5.58 (dd, 1 H, H-1), 5.52 (dd~t, 1 H, H-4), 5.31 (dd, 1H, H-3), 5.24 (dd~t, 1H, H-2), 5.16-5.08 (m, 4H, 2x-CH₂-Ph), 4.07-3.99 (m, 2H, H-5, H-6a), 3.89 (dd, 1H, H-6b), 1.25, 1.21, 1.14, 1.12 (4xs, 36H, 4x-C(CH₃)₃); J_{1,2} = 1.9, J_{2,3} = 3.2, J_{3,4} = 10.4, J_{4,5} = 10.1, J_{5,6a} = 2.8, J_{5,6b} = 1.3, J_{6,6} = 12.6, J_{H-1,P} = 6.3 Hz; ¹³C NMR (100.62 MHz, CDCl₃) δ 178.0, 176.6, 176.4, 172.0 (C=O), 133.7-127.5 (C_{arom.}), 95.6 (d, C-1), 70.6 (C-5), 70.1 (d, -CH₂-Ph), 69.9 (d, -CH₂-Ph), 68.7 (C-3), 68.6 (d, C-2), 64.2 (C-4), 61.0 (C-6), 38.9, 38.8 (Cq, -C(CH₃)₃), 27.2, 27.1 (-C(CH₃)₃); ²J_{C-1,P} = 5.6, 2x ²J_{CH2,P} = 5.6, ³J_{C-2,P} = 11.7 Hz; ³¹P NMR (101.26 MHz, CDCl₃) δ - 1.76; MALDI-TOF-MS: m/z 799.53 [M+Na]⁺, 815.46 [M+K]⁺; Anal. ber. für C₄₀H₅₇O₁₃P (776.86): C 61.84, H 7.40; gefunden: C 61.11, H 7.35.

### Dibenzyl-(2,3,4,6-tetra-O-iso-propylcarbonate-α-D-mannopyranosyl)-phosphat (6).

Die Verbindung 3 (1.19 g, 2.27 mmol) wurde auf die oben beschriebene Weise umgesetzt.

1.54 g (1.96 mmol, 87 %, Sirup); [α]_{D} +6.7 (c 0.5, CHCl₃); R_{f} 0.40 in 1:1 Petrolether/Ethylacetat; ¹H NMR (400 MHz, CDCl₃) δ 7.37-7.32 (m, 10H, Ph), 5.75 (dd, 1H, H-1), 5.23 (dd~t, 1H, H-2), 5.14-5.07 (m, 6H, H-3, H-4, 2x-CH₂-Ph), 4.86 (m, 4H, 4x-CH(CH₃)₂), 4.26 (dd, 1 H, H-6a), 4.18-4.11 (m, 2H, H-5, H-6b), 1.33-1.23 (m, 24H, 8x-CH(CH₃)₂); J_{1,2} = 1.6, J_{2,3} = 2.2, J_{5,6a} = 5.7, J_{6,6} = 11.7, J_{H-1,P} = 6.6 Hz; ¹³C NMR (100.62 MHz, CDCl₃) δ 154.3, 153.5, 153.4 (C=O), 128.7-128.1 (C_{arom.}), 94.9 (d, C-1), 73.3, 73.1, 72.8, 72.3 (-CH(CH₃)₂), 71.7 (C-3), 71.5 (d, C-2), 70.2 (C-5), 70.0 (d, -CH₂-Ph), 69.8 (d, -CH₂-Ph), 69.1 (C-4), 65.3 (C-6), 21.7-21.6 (-CH(CH₃)₂); ²J_{C-1,P} = 5.6, 2x ²J_{CH2,P} = 5.6, ³J_{C-2,P} = 11.7 Hz; ³¹P NMR (101.26 MHz, CDCl₃) δ -1.90; MALDI-TOF-MS: m/z 807.44 [M+Na]⁺, 823.39 [M+K]⁺; Anal. ber. für C₃₆H₄₉O₁₇P (784.76): C 55.10, H 6.29; gefunden: C 55.23, H 6.45.

### Hydrierung

Pd/C (10 %) wurde kontinuierlich zu einer Lösung der Mannopyranosylphosphat Derivate 4, 5 oder 6 in Ethylacetat/Methanol/Wasser (1:2:1) gegeben. Die Mischung wurde bei Raumtemperatur unter H₂-Atmosphäre (50 bar) gerührt. Nach Reaktionsende wurde die Lösung über Celit filtriert und bei vermindertem Druck konzentriert. Der Rest wurde säulenchromatographisch mit Chloroform/Methanol/Wasser (6:3:5:0,5) gereinigt, wobei die Verbindungen 7, 8 oder 9 erhalten wurden.

### 2,3,4,6-Tetra-O-butyryl-α-D-mannopyranosyl phosphat (7).

Die Verbindung 4 (2.43 g, 3.37 mmol) wurde auf die oben beschriebene Weise in 40 ml Lösungsmittel während eines Zeitraumes von 5h umgesetzt.

Ausbeute: 1.35 g (2.50 mmol, 74 %, gelber Sirup); [α]_{D} +37.3 (c 1.0, CHCl₃); R_{f} 0.27 in 6:3.5:0.5 Chloroform/Methanol/Wasser; ¹H NMR (400 MHz, CDCl₃) δ 5.60 (bs, 1H, H-1), 5.40 (dd, 1H, H-3), 5.36 (bs, 1H, H-2), 5.17 (dd-t, 1H, H-4), 4.27-4.13 (m, 1H, H-5), 3.79-3.62 (m, 2H, H6a, H-6b), 2.41-2.24 (m, 8H, 4x-CO-CH₂-), 1.71-1.53 (m, 8H, 4x-CH,₂-CH₃), 1.0-0.87 (m, 12 H, 4x-CH₃); J_{2,3} = 3.6, J_{3,4} = 9.7, J_{4,5} = 10.2 Hz; ¹³C NMR (100.62 MHz, CDCl₃) δ 174.9-172.5 (C=O), 94.9 (bs, C-1), 72.2 (C-5), 69.1 (C-2), 68.5 (C-3), 65.8 (C-4), 61.6 (C-6), 36.0, 35.9 (-CO-CH₂-), 18.4, 18.3, 18.1 (-CH₂-CH₃), 13.5 (-CH₃); ³J_{C-2,P} = 9.2 Hz; ³¹P NMR (101.26 MHz, CDCl₃) δ -1.71; MALDI-TOF-MS: m/z 563.61 [M+Na]⁺, 579.50 [M+K]⁺, 585.49 [M-H+Na+Na]⁺, 601.41 [M-H+Na+K]⁺; Anal. ber. für C₂₂H₃₇O₁₃P (540.50): C 48.89, H 6.90; gefunden: C 48.90, H 6.60.

### . 2,3,4,6-Tetra-O-pivaloyl-α-D-mannopyranosyl phosphat (8).

Die Verbindung 5 (1.30 g, 1.67 mmol) wurde auf die oben beschriebene Weise in 32 ml Lösungsmittel während eines Zeitraumes von 6h umgesetzt.

Ausbeute: 0.82 g (1.37 mmol, 82 %, weißer Feststoff); [α]_{D} +27.0 (c 1.0, CHCl₃); Fp - 245 °C Zersetzung; R_{f} 0.34 in 6:3.5:0.5 Chloroform/Methanol/Wasser; ¹H NMR (400 MHz, Methanol) δ 5.58 (dd~t, 1H, H-4), 5.48 (dd~t, 2H, H-1, H-3), 5.32 (bs, 1H, H-2), 4.48-4.15 (m, 3H, H-5, H-6a, H-6b), 1.28, 1.23, 1.15, 1.10 (4xs, 36H, 4x-C(CH₃)₃); J_{3,4} = 10.4, J_{4,5} = 10.1 Hz; ¹³C NMR (100.62 MHz, Methanol-d₄) δ 179.8, 179.3, 178.8 (C=O), 95.2 (bs, C-1), 71.7 (C-2), 71.4 (C-3), 70.8 (C-5), 66.5 (C-4), 62.9 (C-6), 40.3, 40.2, 40.1, 40.0 (Cq, -C(CH₃)₃), 28.0, 27.9, 27.8 (-C(CH₃)₃; ³J_{C-2,P} = 12.7 Hz; ³¹P NMR (101.26 MHz, Methanol-d₄) δ - 1.75; MALDI-TOF-MS: m/z 619.42 [M+Na]⁺, 635.35 [M+K]⁺, 641.40 [M-H+Na+Na]⁺, 657.33 [M-H+Na+K]⁺, 673.29 [M-H+K+K]⁺; Anal. ber. für C₂₆H₄₅O₁₃P (596.61): C 52.34, H 7.60; gefunden: C 45.28, H 6.61 (hygroskopisches Material).

### 2,3,4,6-Tetra-O-iso-propylcarbonate-α-D-mannopyranosyl phosphat (9).

Die Verbindung 6 (0.45 g, 0.57 mmol) wurde auf die oben beschriebene Weise in 8 ml Lösungsmittel über Nacht umgesetzt.

Ausbeute: 0.26 g (0.43 mmol, 75 %, Feststoff); [α]_{D} +23.4 (c 1.0, CHCl₃); mp 184.1 °C; R_{f} 0.30 in 6:3.5:0.5 Chloroform/Methanol/Wasser; ¹H NMR (400 MHz, Methanol-d₄) δ 5.58 (d, 1 H, H-1), 5.26 (bs, 1H, H-2), 5.21 (dd, 1H, H-3), 5.12 (dd~t, 1 H, H-4), 4.90-4.79 (m, 4H, 4x-CH(CH₃)₂), 4.36-4.21 (m, 3H, H-5, H-6a, H-6b), 1.23-1.20 (m, 24H, 8x-CH(CH₃)₂); J_{2,3} = 3.1, J_{3,4} = 10.2, J_{4,5} = 9.9, J_{H-1,P} = 7.1 Hz; ¹³C NMR (100.62 MHz, Methanol-d₄) δ 156.2, 155.5, 155.1 (C=O), 95.1 (d, C-1), 74.4, 74.2, 73.8, (-CH(CH₃)₂), 74.3 (C-3), 74.3 (d, C-2), 70.9 (C-4), 70.4 (C-5), 66.5 (C-6), 22.3, 22.2 (-CH(CH₃)₂); ²J_{C-1,P} = 3.6, ³J_{C-2,P} = 9.7 Hz; ³¹P NMR (101.26 MHz, Methanol-d₄) δ -1.02; MALDI-TOF-MS: m/z 627.35 [M+Na]⁺, 643.29 [M+K]⁺, 649.33 [M-H+Na+Na]⁺, 665.28 [M-H+Na+K]⁺; Anal. ber. für C₂₂H₃₇O₁₇P (604.51): C 43.71, H 6.17; gefunden: C 44.32, H 6.06.

### Bis-acetoxymethyl-(2,3,4,6-tetra-O-butyryl-α-D-mannopyranosyl)-phosphat (10).

Eine Lösung von Mannopyranosyl-1-phosphat 7 (131 mg, 0.24 mmol) im trockenen Acetonitril (3 ml) wurde bis zur Trockene eingedampft. DIPEA (0.2 ml, 1.2 mmol) und trockenes Acetonitril (3 ml) wurde hinzugegeben, und die Lösung wurde erneut eingeengt und dann im Hochvakuum eingeengt. Anschließend wurde trockenes Acetonitril (3 ml), DIPEA (0.41 ml, 2.4 mmol) und Brommethylacetat (0.59 ml, 6.1 mmol) unter Argon hinzugegeben. Die Mischung wurde bei Raumtemperatur über Nacht gerührt. Die Lösungsmittel wurden abgezogen, und der Rest wurde säulenchromatographisch mit Petrolether/Ethylacetat (1:1) gereinigt, wobei die Verbindung 10 als farbloser Sirup (68 mg, 0.1 mmol) in 41 %-iger Ausbeute erhalten wurde;

[α]_{D} +7.5 (c 0.5, CHCl₃); R_{f} 0.29 in 1:1 Petrolether/Ethylacetat; ¹H NMR (400 MHz, CDCl₃) δ 5.73-5.64 (m, 5H, H-1, 2x-CH₂-, AM), 5.41 (dd~t, 1H, H-4), 5.38 (dd-t, 1H, H-2), 5.35 (dd, 1 H, H-3), 4.28-4.15 (m, 3H, H-5, H-6a, H-6b), 2.42-2.19 (m, 8H, 4x-CO-CH₂-), 2.17, 2.16 (2xs, 6H, -CH₃, AM), 1.75-1.52 (m, 8H, 4x-CH₂-CH₃), 1.04-0.87 (m, 12H, 4x-CH₃); J_{1,2} = 1.9, J_{2,3} = 3.2, J_{3,4} = 9.9, J_{4,5} = 9.9, J_{5,6a} = 3.8, J_{5,6b} = 1.5, J_{6,6} = 11.7, J_{H-1,P} = 7.7 Hz; ¹³C NMR (100.62 MHz, CDCl₃) δ 173.1, 172.2, 172.1 (C=O), 169.3, 169.2 (C=O, AM), 95.9 (d, C-1), 82.7 (dd~t, -CH₂-, AM), 70.8 (C-5), 68.3 (d, C-2), 68.1 (C-3), 64.7 (C-4), 61.4 (C-6), 35.9, 35.8 (-CO-CH₂), 20.6 (-cH₃, AM), 18.4, 18.3, 18.1 (-CH₂-CH₃), 13.7, 13.6, 13.5 (-CH₃); ²J_{C-1,P} = 6.1, ²J_{CH2,P} = 6.1, ³J_{C-2,P} = 12.2 Hz; ³¹P NMR (101.26 MHz, CDCl₃) δ -5.05; MALDI-TOF-MS: mlz 707.29 [M+Na]⁺, 723.19 [M+K]⁺; Anal. ber. für C₂₈H₄₅O₁₇P (684.51): C 49.12, H 6.63; gefunden: C 49.60, H 6.79.

### Bis-pivaloyloxymethyl-(2,3,4,6-tetra-O-butyryl-α-D-mannopyranosyl)-phosphat (11).

Mannopyranosyl-1-phosphat 7 (111 mg, 0.21 mmol) wurde in trockenem Acetonitril (1 ml) suspendiert. DIPEA (0.11 ml, 0.62 mmol) und lodmethylpivaloat (0.15 g, 0.62 mmol) wurden hinzugegeben. Die Mischung wurde über Nacht bei Raumtemperatur gerührt; dann wurde das Lösungsmittel entfernt und der Rückstand in Ethylacetat gelöst. Die Mischung wurde zweimal mit gesättigter Kochsalzlösung gewachsen, über Natriumsulfat getrocknet, filtriert und bei vermindertem Druck eingeengt. Die Reinigung des Produktes erfolgte säulenchromatographisch (Petrolether/Ethylacetat + 1 % Et₃N, 1:1), wobei die Verbindung 11 in 24 %-iger Ausbeute (39 mg, 0.05 mmol, Sirup) erhalten wurde.

[α]_{D} +4.1 (c 0.4, CHCl₃); R_{f} 0.88 in 1:1 Petrolether/Ethylacetat; ¹H NMR (400 MHz, CDCl₃) δ 5.67-5.58 (m, 5H, H-1, 2x-CH₂-, POM), 5.34 (dd~t, 1H, H-4), 5.31 (bs, 1H, H-2), 5.29 (dd, 1H, H-3), 4.22-4.07 (m, 3H, H-5, H-6a, H-6b), 2.33 (dt, 2H, -CO-CH₂-), 2.27 (t, 2H, -CO-CH₂-), 2.19 (dt, 2H, -CO-CH₂-), 2.12 (dt, 2H, -CO-CH₂), 1.68-1.46 (m, 8H, 4x-CH₂-CH₃), 1.18 (s, 18H, -C(CH₃)₃, POM), 0.96-0.81 (m, 12H, 4x-CH₃); J_{1,2} = 1.5, J_{2,3} = 3.1, J_{3,4} = 9.2, J₄,₅ = 9.7, J_{5,6a} = 4.1, J_{6,6} = 12.2 Hz; ¹³C NMR (100.62 MHz, CDCl₃) δ 173.5, 172.6, 172.5, 172.4 (C=O), 96.4 (d, C-1), 83.0 (d, - CH₂-, POM), 82.8 (d, -CH₂-, POM), 70.7 (C-5), 68.3 (d, C-2), 68.1 (C-3), 64.7 (C-4), 61.4 (C-6), 39.1 (Cq, -C(CH₃)₃, POM), 36.4, 36.2 (-CO-CH₂-), 27.2 (-C(CH₃)₃, POM), 18.8, 18.7, 18.5 (-CH₂-CH₃), 14.1, 14.0, 13.9 (-CH₃); ²J_{C-1,P} = 6.1, 2x ²J_{CH2,P} = 6.1, ³J_{C-2,P} = 12.2 Hz; ³¹P NMR (101.26 MHz, CDCl₃) δ -4.92; MALDI-TOF-MS: m/z 791.32 [M+Na]⁺, 807.29 [M⁺K]⁺; Anal. ber. für C₃₄H₅₇O₁₇P (768.81): C 53.12, H 7.47; gefunden: C 53.75, H 7.56.

### Bis-acetoxymethyl-(2,3,4,6-tetra-O-pivaloyl-α-D-mannopyranosyl)-phosphat (12).

Mannopyranosyl-1-phosphat 8 (269 mg, 0.45 mmol) wurde in trockenem Acetonitril (3 ml) und trockenem Toluol (0.5 ml) suspendiert. DIPEA (0.22 ml, 1.35 mmol) und Brommethylacetat (0.13 ml, 1.35 mmol) wurden hinzugegeben. Die Mischung wurde über Nacht bei Raumtemperatur gerührt, wobei die Umsetzung durch TLC (Petrolether/Ethylacetat, 1:1) überwacht wurde. Nach weiterer Zugabe von Brommethylacetat (0.1 ml, 1.02 mmol) und DIPEA (0.1 ml, 0.59 mmol) wurde die Suspension erneut bei Raumtemperatur für 2 Tage gerührt. Danach wurden die Lösungsmittel entfernt, der Rückstand in Ethylacetat (5 ml) und Dichlormethan (5 ml) gelöst. Die Mischung wurde zweimal mit gesättigter Kochsalzlösung gewaschen, über Natriumsulfat getrocknet, filtriert und bei vermindertem Druck eingeengt. Der Rohrückstand wurde säulenchromatographisch (Petrolether/Ethylacetat, 2:1) gereinigt, wobei die Verbindung 12 (104 mg, 0.14 mmol) in 31 %-iger Ausbeute als gelblicher Feststoff erhalten wurde.

[α]_{D} +12.0 (c 0.5, CHCl₃); mp 88.5 °C; R_{f} 0.31 in 1:1 Petrolether/Ethylacetat; ¹H NMR (400 MHz, CDCl₃) δ 5.74-5.63 (m, 5H, H-1, 2x-CH₂-, AM), 5.58 (dd-t, 1H, H-4), 5.39-5.35 (m, 2H, H-2, H-3), 4.29-4.16 (m, 3H, H-5, H-6a, H-6b), 2.17, 2.16 (2xs, 6H, -CH₃, AM), 1.28, 1.24, 11.16, 1.12 (4xs, 36H, 4x-C(CH₃)₃); J_{1,2} = 1.8, J_{3,4} = 9.4, J_{4,5} = 10.2 Hz; ¹³C NMR (100.62 MHz, CDCl₃) δ 178.0, 176.4 (C=O), 171.2, 169.2, 169.1 (C=O, AM), 96.2 (d, C-1), 82.7 (d, -CH₂-, AM), 82.6 (d, -CH₂-, AM), 71.0 (C-5), 68.6 (C-3), 68.4 (d, C-2), 64.1 (C-4), 61.1 (C-6), 38.9, 38.8, 38.7 (Cq, -C(CH₃)₃), 27.1, 27.0 (-C(CH₃)₃), 21.1, 20.6, 20.5 (-CH₃, AM); ²J_{C-1,P} = 5.1, 2x ²J_{CH2,P} = 5.1 , ³J_{C-2,P} = 12.2 Hz; ³¹p NMR (101.26 MHz, CDCl₃) δ -5.47; MALDI-TOF-MS: m/z 763.52 [M+Na]⁺, 779.46 [M+K]⁺; Anal. ber. für C₃₂H₅₃0₁₇P (740.74): C 51.89, H 7.21; gefunden: C 51.19, H 7.32.

### Bis-pivaloyloxymethyl-(2,3,4,6-tetra-O-pivaloyl-α-D-mannopyranosyl)-phosphat (13).

Die Verbindung 8 (232 mg, 0.39 mmol) wurde auf die gleiche Weise [trockenes Acetonitril (3 ml), trockenes Toluol (1 ml), lodmethylpivaloat (0.57 g, 2.34 mmol), DIPEA (0.40 ml, 2.34 mmol), gerührt für 3 Tage] wie bei der Verbindung 11 behandelt. Der erhaltene Rückstand wurde säulenchromatographisch (Petrolether/Ethylacetat + 1 % Et₃N, 3:1) gereinigt, wobei die Verbindung 13 als weißer Feststoff (18 mg, 0.02 mmol) in 6 %-iger Ausbeute erhalten wurde.

[α]_{D} +6.1 (c 0.5, CHCl₃); mp 86.7 °C; R_{f} 0.62 in 1:1 Petrolether/Ethylacetat; ¹H NMR (400 MHz, CDCl₃) δ 5.75-5.64 (m, 5H, H-1, 2x-CH₂-POM), 5.58 (dd-t, 1H, H-4), 5.41-5.36 (m, 2H, H-2, H-3), 4.32-4.22 (m, 2H, H-5, H-6a), 4.13 (d, 1H, H-6b), 1.27, 1.23, 1.16, 1.11 (4xs, 36H, 4x-C(CH₃)₃), 1.24 (2xs, 18H, 2x-C(CH₃)₃, POM); J_{1,2} = 1.5, J_{2,3} = 3.1, J_{3,4} = 9.9, J_{4,5} = 9.9, J_{5,6a} = 2.8, J_{6,6} = 11.2 ³J_{H-1,P} = 5.6 Hz; ¹³C NMR (100.62 MHz, CDCl₃) δ 177.9, 176.9, 176.7, 176.5 (C=O), 96.2 (d, C-1), 83.0 (d, -CH₂-, POM), 82.9 (d, -CH₂-, POM), 70.9 (C-5), 68.5 (d, C-2), 68.5 (C-3), 64.2 (C-4), 61.2 (C-6), 38.9, 38.8, 38.7 (Cq, -C(CH₃)₃, Piv, POM), 27.1, 27.0 (-C(CH₃)₃), 26.8 (-C(CH₃)₃, POM); ²J_{C-1,P} = 5.6, 2x ²J_{CH2,P} = 5.1 ³J_{C-2,P} = 12.7 Hz; ³¹P NMR (101.26 MHz, CDCl₃) δ -5.38; MALDI-TOF-MS: m/z 847.33 [M+Na]⁺, 863.30 [M+K]⁺; Anal. ber. für C₃₈H₆₅O₁₇P (824.90): C 55.33, H 7.94; gefunden: C 56.10, H 7.99.

### Bis-acetoxymethyl-(2,3,4,6-tetra-O-iso-propylcarbonate-α-D-mannopyranosyl)-phosphat (14).

Mannopyranosyl-1-phosphat 9 (25.6 mg, 0.04 mmol) wurde auf die gleiche Weise [trockenes Acetonitril (1 ml + 1 ml + 0.5 ml), DIPEA (0.02 ml, 0.12 mmol + 0.04 ml, 0.24 mmol), Brommethylacetat (96 µl, 0.98 mmol), für 3 Tage gerührt, säulenchromatographisch mit Petrolether/Ethylacetat (1:1)] wie im Falle der Verbindung 10 behandelt.

Es wurde die Verbindung 14 (5.4 mg, 7.2 µmol) als farbloser Sirup in einer Ausbeute von 17 % erhalten.

[α]_{D} -1.6 (c 0.6, CHCl₃); R_{f} 0.22 in 1:1 Petrolether/Ethylacetat; ¹H NMR (400 MHz, CDCl₃) δ 5.79 (dd, 1H, H-1), 5.73-5.64 (m, 4H, 2x-CH₂-, AM), 5.51 (dd~t, 1H, H-2), 5.16-5.13 (m, 2H, H-3, H-4), 4.92-4.81 (m, 4H, 4x-CH(CH₃)₂), 4.33 (dd, 1H, H-6a), 4.28-4.22 (m, 2H,H-5, H-6b), 2.18, 2.16 (2xs, 6H, -CH₃, AM), 1.34-1.25 (m, 24H, 4x-CH(CH₃)₂); J_{1,2} = 1.5, J_{2,3} = 2.0, J_{5,6a} = 6.4, J_{6,6} = 12.2 Hz; ¹³C NMR (100.62 MHz, CDCl₃) δ 168.3, 168.2 (C=O, AM), 154.2, 153.5, 153.4, 153.3 (C=O), 95.5 (d, C-1), 82.8 (d, -CH₂-, AM), 82.7 (d, -CH₂-, AM), 73.5, 73.2, 72.9, 72.4 (-CH(CH₃)₂), 71.5, 68.9 (C-3, C-4), 71.2 (d, C-2), 70.5 (C-5), 65.2 (C-6), 21.7, 21.6, 21.5 (-CH(CH₃)₂), 20.6, 20.5 (-CH₃, AM); ²J_{C-1,P} = 5.1, 2x ²J_{CH2,P} = 5.1, ³J_{C-2,P} = 12.2 Hz; ³¹P NMR (101.26 MHz, CDCl₃) δ -5.30; MALDI-TOF-MS: m/z 771.08 [M+Na]⁺, 787.03 [M+K]⁺; Anal. ber. für C₂₈H₄₅O₂₁P (748.51): C 44.92, H 6.06; gefunden: C 45.09, H 6.20.

### Bis-pivaloyloxy-(2,3,4,6-tetra-O-iso-propylcarbonat-α-D-mannopyranosyl)-phosphat (15).

Die Verbindung 9 (189 mg, 0.31 mmol) wurde in Acetonitril (3 ml), DIPEA (0.16 ml, 0.94 mmol) suspendiert, und lodmethylpivaloat (0.23 g, 0.94 mmol) wurde hinzugegeben. Die Mischung wurde über Nacht bei Raumtemperatur gerührt. Der Reaktionsverlauf wurde mittels TLC (Petrolether/Ethylacetat, 1:1) verfolgt. Die Mischung wurde für weitere 3 Tage nach Zugabe von DIPEA (0.16 ml) und lodmethylpivaloat (0.23 g) gerührt. Die Lösungsmittel wurde entfernt, der Rest wurde in Ethylacetat (5 ml) und Dichlormethan (5ml) gelöst. Die Mischung wurde zweimal mit gesättigter Kochsalzlösung gewaschen, über Natriumsulfat getrocknet, filtriert und bei vermindertem Druck eingeengt. Der Rohrückstand wurde säulenchromatographisch gereinigt (Petrolether/Ethylacetat + 1 % Et₃N, 3:1), wobei die Verbindung 15 (9.3 mg, 0.01 mmol) als farbloser Sirup in 4 %-iger Ausbeute erhalten wurde.

[α]_{D} +2.3 (c 0.5, CHCl₃); R_{f} 0.5 in 1:1 Petrolether/Ethylacetat; ¹H NMR (400 MHz, CDCl₃) δ 5.78 (dd, 1H, H-1 ), 5.75-5.65 (m, 4H, 2x-CH₂-, POM), 5.30 (dd~t, 1H, H-2), 5.16-5.12 (m, 2H, H-3, H-4), 4.91-4.81 (m, 4H, 4x-CH(CH₃)₂), 4.33 (dd, 1H, H-6a), 4.27-4.22 (m, 2H, H-5, H-6b), 1.32-1.26 (m, 24H, 4x-CH(CH₃)₂), 1.24, 1.23 (2xs, 18H, 2x-C(CH₃)₃, POM); J_{1,2} = 1.8, J_{2,3} = 4.9, J_{5,6a} = 6.4, J_{5,6b} = 2.5, J_{6,6} = 12.2, J_{CH,CH3} = 6.4, ³J_{H-1,P} = 5.6 Hz; ¹³C NMR (100.62 MHz, CDCl₃) δ 95.5 (d, C-1), 83.0 (d, -CH₂-, POM), 82.9 (d, -CH₂-, POM), 73.4, 73.1, 72.9, 72.4 (-CH(CH₃)₂), 71.6 (C-3), 71.3 (d, C-2), 70.5 (C-5), 69.0 (C-4), 65.2 (C-6), 26.8 (-C(CH₃)₃, POM), 21.7, 21.6 (-CH(CH₃)₂); ²J_{C-1,P} = 5.1, 2x ²J_{CH2,P} = 5.1, ³J_{C-2,P} = 12.2 Hz; ³¹P NMR (101.26 MHz, CDCl₃) δ -5.23; MALDI-TOF-MS: m/z 855.26 [M+Na]⁺, 871.22 [M+K]⁺; Anal. ber. für C₃₄H₅₇O₂₁P (8.32.79): C 49.04, H 6.90; gefunden: C 50.45, H 7.34.

## Patentansprüche

1. Mannose 1-Phosphat Derivate der folgenden allgemeinen Formel I 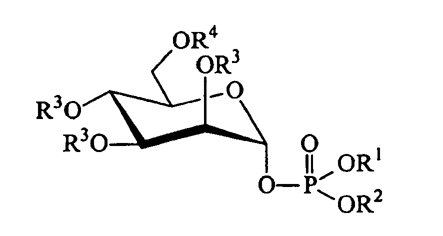
worin
R¹ und R², die gleich oder verschieden sein können, für
H oder 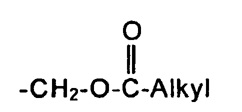 stehen,
oder R¹ und R² zusammen für: 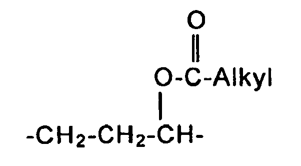 stehen,
mit der Maßgabe, dass nur einer der Reste R¹ und R² für H stehen kann, und
R³ und R⁴, die gleich oder verschieden sein können, für 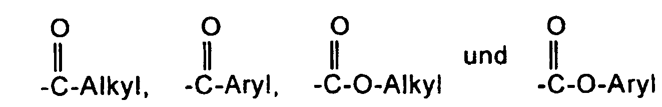 stehen
und der Rest R³ auch für H stehen kann, wobei
Aryl einen aromatischen Kohlenwasserstoffrest bedeutet, der durch einen Rest Alkyl substituiert sein kann, und
Alkyl einen linearen oder verzweigten, gesättigten Kohlenwasserstoffrest mit 1 bis 20 Kohlenstoffatomen bedeutet.

2. Mannose 1-Phosphat Derivate nach Anspruch 1,
**dadurch gekennzeichnet,**
**dass** in der allgemeinen Formel I die Reste R¹ und R² gleich sind, alle Reste ³ gleich sind und/oder alle Reste R³ und R⁴ gleich sind.

3. Mannose 1-Phosphat Derivate nach Anspruch 1 oder 2,
**dadurch gekennzeichnet,**
**dass** der Rest Alkyl in einem oder mehreren der Reste R¹ bis R⁴ für -CH₃, -C(CH₃)₃, -CH(CH₃)₂ und -CH₂-CH₂-CH₃ steht.

4. Pharmazeutisches oder diätetisches Mittel,
**dadurch gekennzeichnet,**
**dass** es mindestens ein Mannose 1-Phosphat Derivat nach einem der Ansprüche 1 bis 3 enthält oder daraus aufgebaut ist.

5. Pharmazeutisches oder diätetisches Mittel nach Anspruch 4 zur Behandlung von angeborenen Glykosylierungsstörungen.

6. Verwendung von Mannose 1-Phosphat Derivaten der Formel I gemäß Anspruch 1, in der die Reste R¹, R², R³ und R⁴ physiologisch vorkommende Carbonsäuregruppen, die als Ester gebunden sind, bedeuten und R³ auch ein H-Atom bedeuten kann, zur Herstellung von pharmazeutischen und diätetischen Mitteln zur Behandlung von angeborenen Glykosylierungsstörungen.

7. Verwendung nach Anspruch 6, wobei mindestens ein Mannose 1-Phosphat Derivat nach einem der Ansprüche 1 bis 3 eingesetzt wird.

8. Verwendung nach Anspruch 6 oder 7 zur Herstellung von pharmazeutischen und diätetischen Mitteln zur Behandlung von CDG-Ia.

## Claims

1. Mannose 1-phosphate derivatives of the following general formula (I) 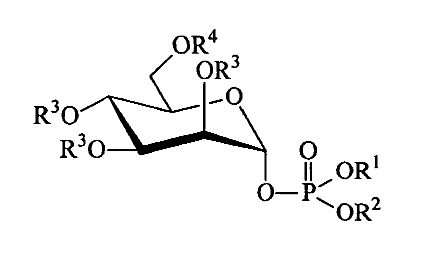
wherein
R¹ and R², which may be the same or different, represent
H or 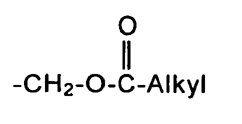
or R¹ and R² represent together: 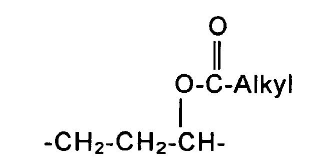
with the proviso that only one of the radicals R¹ and R² is H, and
R³ and R⁴, which may be the same or different, represent 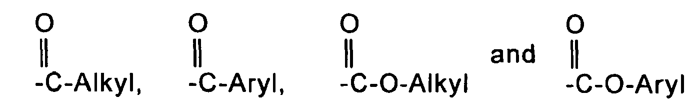
and the radical R³ can also represent H whereby
Aryl represents an aromatic hydrocarbon radical which can be substituted by the radical alkyl, and,
Alkyl represents a linear or branched, saturated hydrocarbon radical having 1 to 20 carbon atoms.

2. Mannose 1-phosphate derivatives according to claim 1,
**characterized in that**
the radicals R¹ and R² in the general formula I are the same, all radicals R³ are the same and/or all radicals R³ and R⁴ are the same.

3. Mannose 1-phosphate derivatives according to claim 1 or 2,
**characterized in that**
the radical Alkyl in one or more of the radicals R¹ to R⁴ represents -CH₃, -C(CH₃)₃, -CH(CH₃)₂ and -CH₂-CH₂-CH₃.

4. Pharmaceutical or dietetical composition,
**characterized in that**
it contains at least one mannose 1-phosphate derivative according to one of the claims 1 to 3 or is composed thereof.

5. Pharmaceutical or dietetical composition according to claim 4 for the treatment of inborn disorders of glycosylation.

6. Use of mannose 1-phosphate derivatives of the formula I according to claim 1 in which the radicals R¹, R², R³ and R⁴ represent physiologically occurant carbonic acid groups which are bound in the form as an ester, and R³ can also represent a H-atom, for the production of pharmaceutical and dietetical compositions for the treatment of inborn disorders of glycosylation.

7. Use according to claim 6, whereby at least one mannose 1-phosphate derivative according to one of the claims 1 to 3 is used.

8. Use according to claim 6 or 7 for the production of a pharmaceutical and dietetical composition for the treatment of CDG-Ia.

## Revendications

1. Dérivés de mannose 1-phosphate de formule générale I suivante 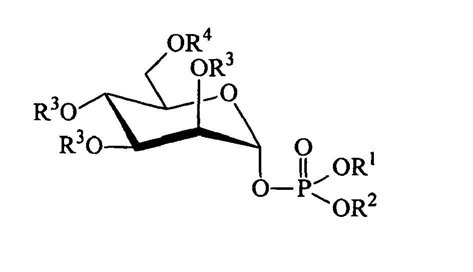
où
R¹ et R², qui peuvent être semblables ou différents, figurent pour
H ou 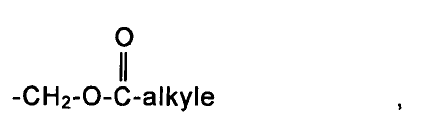
ou R¹ et R² ensemble figurent pour : 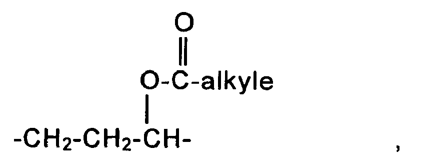
étant déterminant que seul l'un des restes R¹ et R² peut figurer pour H et
R³ et R⁴, qui peuvent être semblables ou différents, figurent pour 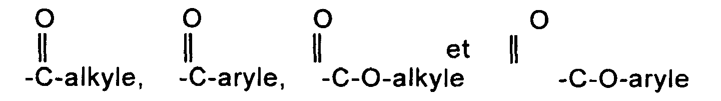
et le reste R³ pouvant également figurer pour H,
aryle signifiant un reste d'hydrocarbure aromatique pouvant être remplacé par un reste d'alkyle, et
alkyle signifiant un reste d'hydrocarbure saturé linéaire ou ramifié, avec 1 à 20 atomes de carbone.

2. Dérivés de mannose 1-phosphate selon la revendication 1,
**caractérisés en ce que**
dans la formule générale I, les restes R¹ et R² sont égaux, tous les restes ³ sont égaux et/ou tous les restes R³ et R⁴ sont égaux.

3. Dérivés de mannose 1-phosphate selon la revendication 1 ou 2,
**caractérisés en ce que**
l'alkyle restant figure dans un ou plusieurs des restes R¹ à R⁴ pour -CH₃, -C(CH₃)₃, -CH(CH₃)₂ et -CH₂-CH₂-CH₃.

4. Produit pharmaceutique ou diététique,
**caractérisé en ce que**
il contient au moins un dérivé de mannose 1-phosphate selon l'une quelconque des revendications 1 à 3 ou est élaboré à partir de lui.

5. Produit pharmaceutique ou diététique selon la revendication 4 pour le traitement de troubles congénitaux de la glycolisation.

6. Utilisation de dérivés de mannose 1-phosphate de formule I selon la revendication 1, dans laquelle les restes R¹, R², R³ et R⁴ signifient des groupes d'acide carboxylique pouvant se produire physiologiquement, liés en tant qu'ester, et R³ peut également signifier un atome H, pour la fabrication de produits pharmaceutiques et diététiques pour le traitement de troubles congénitaux de la glycolisation.

7. Utilisation selon la revendication 6, dans laquelle au moins un dérivé de mannose 1-phosphate selon l'une quelconque des revendications 1 à 3 est employé.

8. Utilisation selon la revendication 6 ou 7 pour la fabrication de produits pharmaceutiques et diététiques pour le traitement du CDG-la.
